# EUROPÄISCHE PATENTSCHRIFT

(11) **EP 2 140 910 B1**
(45) Veröffentlichungstag und Bekanntmachung des Hinweises auf die Patenterteilung: **15.04.2015**
(21) Anmeldenummer: 09160086.6
(22) Anmeldetag: 13.05.2009
(51) Int. Cl.: A61N 1/39, A61N 1/362, A61B 5/0464

(54) **Herzstimulator zur Behandlung tachykarder Rhythmusstörungen eines Herzens**
Heart stimulator for treating cardiac tachyarrhythmias
Stimulateur cardiaque destiné au traitement des rythmes tachycardiques

(30) Priorität: 11.06.2008 DE 102008002370
(43) Veröffentlichungstag der Anmeldung: 06.01.2010
(73) Patentinhaber: Biotronik CRM Patent AG, 6341 Baar (CH)
(72) Erfinder: Dörr, Thomas, 12437, Berlin (DE)
(74) Vertreter: Lindner-Vogt, Karin L.

(56) Entgegenhaltungen:
- EP-A- 1 774 988
- WO-A-97/36647
- WO-A-2004/103172
- US-A- 5 193 550
- US-A1- 2005 149 135
- US-A1- 2007 038 253

## Beschreibung

Die Erfindung betrifft einen implantierbaren Herzstimulator, der als Cardioverter/Defibrillator (ICD) zur Abgabe eines Defibrillationsschocks im Falle einer ventrikulären Fibrillation ausgebildet ist.

Implantierbare Herzstimulatoren in Form von Herzschrittmachern oder Cardiovertern/Defibrillatoren sind grundsätzlich bekannt. Solche Herzstimulatoren sind in der Regel an Elektrodenleitungen angeschlossen, die in einer Kammer eines Herzens oder in unmittelbarer Nähe Stimulations- und optional zusätzliche Defibrillationselektroden besitzen. Über eine Stimulationselektrode kann ein Herzschrittmacher einen elektrischen Stimulationsimpuls an das Muskelgewebe einer Herzkammer abgeben, um so eine stimulierte Kontraktion der Herzkammer hervorzurufen, sofern der Stimulationsimpuls eine ausreichende Intensität besitzt und das Herzmuskelgewebe (Myokard) sich nicht gerade in einer refraktären Phase befindet. Um auf diese Weise eine stimulierte Kontraktion einer Herzkammer auszulösen, werden üblicherweise Elektrodenleitungen mit relativ kleinflächigen Stimulationselektroden verwendet, da es zum Auslösen einer stimulierten Kontraktion einer Herzkammer ausreicht, wenn nur ein kleiner Teil des Myokards dieser Herzkammer anfänglich stimuliert wird. Eine derartige stimulierte Kontraktion einer Herzkammer wird im Rahmen dieser Beschreibung als stimuliertes Ereignis bezeichnet. Kommt es zu einer natürlichen Kontraktion der Herzkammer, wird dies im Rahmen dieser Beschreibung als Eigenaktion oder als intrinsisches Ereignis bezeichnet. Eine Kontraktion beispielsweise des rechten Atriums eines Herzens wird als atriales Ereignis bezeichnet, welches beispielsweise ein natürliches atriales Ereignis sein kann, oder - im Falle eines atrialen Herzschrittmachers - auch ein stimuliertes atriales Ereignis. Im gleichen Sinne können natürliche (intrinsische) und stimulierte linksventrikuläre und rechtsventrikuläre Ereignisse unterschieden werden.

Eine lokale Erregung des Myokards breitet sich vom Erregungsort ausgehend per Reizleitung im Myokard aus und führt zu einer Depolarisation der Muskelzellen und damit zu einer Kontraktion des Myokards. Nach kurzer Zeit kommt es zu einer Repolarisation der Muskelzellen und damit zu einer Entspannung des Myokards. Während der Phase der Depolarisation sind die Herzmuskelzellen für eine Erregung unempfindlich, d.h. refraktär. Die mit der Depolarisation und Repolarisation einhergehenden elektrischen Potentiale können wahrgenommen und deren zeitlicher Verlauf - als Elektrokardiogramm bezeichnet - ausgewertet werden.

Das Erfassen solcher natürlichen (intrinsischen) Ereignisse erfolgt durch Ableiten der elektrischen Potenziale des Myokards der jeweiligen Herzkammer mit Hilfe von Sensingelektroden, die Teil einer entsprechenden Elektrodenleitung sind. Dabei können die Sensingelektroden gleichzeitig die Stimulationselektroden sein und abwechselnd als Stimulations- und als Sensingelektroden verwendet werden. Typischerweise ist für das Sensing - d.h. die Wahrnehmung intrinsischer Ereignisse - ein Sensingelektrodenpaar vorgesehen, das von zwei benachbarten Elektroden, nämlich einer Spitzenelektrode (Tip-Elektrode) und einer Ringelektrode gebildet ist, von denen die Spitzenelektrode auch als Stimulationselektrode dient. Auf diese Weise erfolgt eine bipolare Ableitung eines intrakardialen Elektrokardiogramms (IEGM). Dabei erfolgt das Sensing und die Stimulation im Ventrikel mit Hilfe einer ventrikulären Elektrodenleitung und die Stimulation und das Sensing im Atrium (im rechten Atrium) mit einer atrialen Elektrodenleitung, die separat an den jeweiligen Herzstimulator angeschlossen sind. Zusätzlich kann auch eine linksventrikuläre Elektrodenleitung vorgesehen sein, die typischerweise über den Coronarsinus und eine von diesem abzweigende Lateralvene in die Nähe des linken Ventrikels ragt und dort eine kleinflächige Stimulations- und/oder Sensingelektrode aufweisen kann.

Die Sensingelektroden sind im Betrieb des Herzstimulators mit entsprechenden Sensingeinheiten verbunden, die ausgebildet sind, ein jeweiliges über eine Sensingelektrode (bzw. ein Sensingelektrodenpaar) aufgenommenes Elektrokardiogramm auszuwerten und insbesondere intrinsische atriale bzw. ventrikuläre Ereignisse zu detektieren, d.h. natürliche atriale oder ventrikuläre Kontraktionen. Dies geschieht beispielsweise durch Schwellwertvergleich, d.h. ein intrinsisches Ereignis wird detektiert, wenn ein jeweiliges intrakardiales Elektrokardiogramm einen geeignet vorgegebenen Schwellwert überschreitet.

Aus der Frequenz, mit der atriale bzw. ventrikuläre Ereignisse aufeinander folgen, kann die jeweilige intrinsische atriale Herzrate (atriale Frequenz) bzw. ventrikuläre Herzrate (Ventrikelfrequenz) abgeleitet und somit beispielsweise Tachykardien detektiert werden.

Es sind auch Herzstimulatoren bekannt, z.B. aus WO 2004/103172, US2007/0038253 und US5193550, die zur Besseren Detektion atrialer und/oder ventrikulärer Ereignisse jeweils mehrere unabhängige Sensingeinheiten vorsehen. Ein implantierbarer Cardioverter/Defibrillator besitzt über die bereits beschriebenen Eigenschaften eines Herzschrittmachers hinaus weiterhin die Möglichkeit, einen stärkeren Stromimpuls an das Herz abzugeben, der nicht nur einen kleinen Teil des Myokards stimulieren (depolarisieren) soll, sondern einen möglichst großen Teil des Myokards depolarisieren und damit refraktär machen soll, um so eine für Fibrillationen typische kreisende Erregung des Myokards zu unterbrechen. Ein derartiger Impuls wird als Defibrillations-schock bezeichnet. Er wird typischerweise über eine im Vergleich zur Stimulations- oder Sensingelektrode großflächige Defibrillationselektrode abgegeben.

Solch eine Defibrillationselektrode ist häufig in Form einer Schockwendel auf der Außenfläche der Elektrodenleitung in der jeweiligen Herzkammer realisiert. Beispielsweise kann eine ventrikuläre Elektrodenleitung neben einer Tip-Elektrode oder einer Ringelektrode für die Stimulation und das Sensing auch eine ventrikuläre Schockwendel und darüber hinaus auch eine nach Implantation in der Vena cava superior befindliche proximale Schockwendel besitzen.

Die Abgabe eines Defibrillationsschocks erfolgt in der Regel dann, wenn der Herzstimulator eine Fibrillation, d.h. eine unregelmäßige hochfrequente Eigenaktivität des Herzens, detektiert, die auch als Kammerflimmern bezeichnet wird und die dazu führt, dass es zu keiner vollständigen Kontraktion der betroffenen Herzkammer kommt. Eine solche Fibrillation zählt zu den tachykarden Arrhythmien, zu denen neben Fibrillationen auch Tachykardien (z.B. Kammerflattern) zählen. Im Unterschied zu einer Fibrillation folgt bei einer Tachykardie regelmäßig eine vollständige Kontraktion der betroffenen Herzkammer, jedoch mit einer Rate, die höher ist als physiologisch angemessen. Häufig können solche Tachykardien durch eine antitachykarde Stimulation behandelt werden und bedürfen nicht eines Defibrillationsschocks. Fibrillationen werden in der Regel mit einem Defibrillationsschock behandelt.

Zur Detektion von Kammerflimmern (ventrikulärer Fibrillation) ist typischerweise eine Detektionseinrichtung als Bestandteil einer Steuereinheit des Herzstimulators vorgesehen, die mit der rechtsventrikulären Sensingeinheit verbunden und ausgebildet ist, eine primäre Kammerflimmerdetektion auf Basis des rechtsventrikulären, bipolar abgeleiteten intrakardialen Elektrokardiogramms (IEGM) durchzuführen. Ist eine vorgegebene Detektionsbedingung (X aus Y-Kriterium) erfüllt, zeigt die Detektionseinrichtung eine ventrikuläre Fibrillation an.

Bei tachykarden Rhythmusstörungen des Ventrikels unterscheidet man zwischen supraventrikulären Tachykardien (SVT) und ventrikulären Tachykardien (VT) im engeren Sinne. Letztere haben ihren Ursprung im Ventrikel selbst, während supraventrikuläre Tachykardien ihren Ursprung im Atrium haben. Für die nach Detektion einer Tachykardie eingeleitete Therapie ist die Art der ventrikulären Tachykardie (ventrikuläre Tachykardie im engeren Sinne (VT) oder supraventrikuläre Tachykardie (SVT)) von Bedeutung.

Die Behandlung von Tachykardien und Fibrillationen mittels intrakardialer Elektrotherapie kann auf verschiedene bekannte Arten und Weisen erfolgen. Bekannt ist eine antitachykarde Stimulation (anti tachycardia pacing, ATP) in Form einer Overdrive-Stimulation, bei der Stimulationsimpulse mit einer gegenüber über der der vorliegenden, intrinsischen (tachykarden) Herzrate erhöhten Stimulationsrate abgegeben werden, die Abgabe von Cardioversionsschocks oder die Abgabe von Defibrillationsschocks, wobei erstere üblicherweise eine geringere Energie haben als letztere. Defibrillationsschocks sollen das gesamte Myokard einer betroffenen Herzkammer gleichzeitig refraktär und damit für eine Erregung temporär unempfänglich machen, um so eine kreisende Erregung des betroffenen Herzmuskels zu unterbrechen.

Die Behandlung ventrikulärer Tachykardien im engeren Sinne (VT) durch Overdrive-Stimulation im Rahmen eines antitachykarden pacings (ATP) soll bewirken, dass durch einen Stimulationsimpuls, der vor der natürlichen (intrinsischen) Erregung der betroffenen Herzkammer erfolgt, ein für ventrikuläre Tachykardien (VT, Kammerflattern) typischer Wiedereintritts-Zyklus (Reentry-Zyklus) der Erregung des Myokards unterbrochen wird. Hierzu ist eine zuverlässige Erfassung intrinsischer Ventrikelkontraktionen vor dem Auslösen der ATP erforderlich.

Das der Erfindung zugrunde liegende Problem besteht darin, dass es unter Umständen zu einem Oversensing ventrikulärer Ereignisse kommen kann, d.h. dazu, dass die entsprechende ventrikuläre Sensingeinheit mehr vermeintlich ventrikuläre Ereignisse detektiert, als tatsächlich vorliegen. Solches Oversensing führt in vielen Fällen zur Abgabe inadäquater Defibrillationsschocks oder zumindest zum Start von Ladevorgängen aufgrund eines wahrgenommenen Störsignals an der rechtsventrikulären Elektrode. Durch diese Ladevorgänge wird die Laufzeit des ICD deutlich reduziert.

Aufgabe der Erfindung ist es, eine inadäquate Abgabe Defibrillationsschocks oder einen unnötigen Start von Ladevorgängen infolge Oversensings möglichst zu vermeiden.

Diese Aufgabe lässt sich durch einen implantierbaren antitachykarden Herzstimulator lösen, der wenigstens aufweist:
- eine rechtsventrikuläre Sensingeinheit zur Wahrnehmung rechtsventrikulärer Ereignisse mittels wenigstens einer Wahrnehmungselektrode (Sensingelektrode) im rechten Ventrikel, mit der die rechtsventrikuläre Sensingeinheit über eine rechtsventrikuläre Elektrodenleitung verbunden ist oder verbunden werden kann,
- einen Defibrillationsschockgenerator, der einen Defibrillationsschock abhängig von der Detektionseinrichtung generieren und über wenigstens einer Defibrillationsschockelektrode abgeben kann,
- eine Steuereinheit, die unter anderem als Detektionseinrichtung zur Detektion eines ventrikulären Kammerflimmerns (VF) auf Basis der rechtsventrikulär wahrgenommenen Ereignisse und zur Ansteuerung des Defibrillationsschockgenerators ausgebildet ist,
sowie
- eine zusätzliche Erfassungseinrichtung zur Wahrnehmung ventrikulärer Ereignisse, die unabhängig von der rechtsventrikulären Wahrnehmungselektrode arbeitet, und
- eine Auswerteeinheit (z.B. als weiteren Bestandteil der Steuereinheit), die bei sicherem Nachweis eines Normalrhythmus mit der zusätzlichen Erfassungseinrichtung die Abgabe eines Defibrillationsschocks inhibiert.

Bei einem biventrikulären Herzstimulator mit einem Anschluss für eine linksventrikuläre Elektrodenleitung, beispielsweise eine Koronarsinus(CS)-Elektrodenleitung, ist die zusätzliche Erfassungseinrichtung vorzugsweise mit der linksventrikulären Elektrodenleitung bzw. deren Anschluss verbunden. Die Auswerteeinheit ist dann dazu ausgebildet, einen Normalrhythmus anhand eines mittels einer linksventrikulären Sensingelektrode zu gewinnenden EKG-Signals zu detektieren. Dazu kann die Auswerteinheit mit einer linksventrikulären Sensingeinheit verbunden und zur Auswertung von linksventrikulären, von der linksventrikulären Sensingeinheit detektierten Sense-Ereignissen ausgebildet sein.

Gemäß der Erfindung ist der Herzstimulator ein Dreikammer-Herzstimulator mit Anschlüssen für eine linksventrikuläre und eine rechtsatriale Elektrodenleitung.

In Verbindung mit der zusätzlichen Erfassungseinrichtung ist die Auswerteeinheit ausgebildet, einen Rhythmus dann als "Normalrhythmus" zu bewerten, wenn nach einem atrialen Ereignis (A-Sense, A-Pace) innerhalb eines vorzugsweise programmierbaren Zeitfensters (AV-Zeiterwartung) ein einziges linksventrikuläres Ereignis wahrgenommen wird UND das Intervall der linksventrikulären Ereignisse dem der vorausgegangenen atrialen Ereignisse zuzüglich einer Toleranz entspricht.

Hierbei ist die AV-Zeiterwartung für die linksventrikuläre Wahrnehmung nach atrialen Ereignissen vorzugsweise für den Arzt einstellbar.

Zusätzlich oder alternativ kann die zusätzliche Erfassungseinrichtung auch zur Ableitung eines Fernfeldelektrokardiogramms, beispielsweise zwischen einer Schockelektrode und einem elektrisch leitenden Gehäuse des ICDs ausgebildet sein.

Hierzu besitzt der implantierbare Herzstimulator vorzugsweise ein Gehäuse, das wenigstens zum Teil eine elektrisch leitende Oberfläche aufweist sowie einen Defibrillationselektrodenleitungsanschluss für den Anschluss einer ventrikulären Defibrillationselektrodenleitung, wobei der Defibrillationselektrodenleitungsanschluss einen elektrischen Kontakt besitzt, der mit einem Gegenkontakt zu verbinden ist, welcher bei an den Herzstimulator angeschlossener Defibrillationselektrodenleitung mit deren Defibrillationselektrode elektrisch verbunden ist. Die zusätzliche Erfassungseinrichtung weist gemäß dieser Ausführungsvariante eine Fernfeldelektrokardiogramm-Erfassungseinheit auf, die einen ersten Eingang aufweist, der vorzugsweise mit einem Anschluss für eine ventrikuläre Defibrillationselektrode verbunden ist, sowie einen zweiten Eingang, der vorzugsweise mit der elektrisch leitenden Oberfläche des Gehäuses des Herzstimulators verbunden ist. Die Fernfeldelektrokardiogramm-Erfassungseinheit ist ausgebildet, auf Basis der an den beiden Eingängen im Betrieb anliegenden elektrischen Potenziale ein Fernfeldelektrokardiogramm zu generieren.

Gemäß dieser Ausführungsvariante weist die zusätzliche Erfassungseinheit außerdem eine Fernfeldelektrokardiogramm-Auswerteeinheit auf, die mit der Fernfeldelektrokardiogramm-Erfassungseinheit verbunden und ausgebildet ist, in einem von der Fernfeldelektrokardiogramm-Erfassungseinheit generierten Fernfeldelektrokardiogramm für ventrikuläre Depolarisationen charakteristische Signalmerkmale, z.B: QRS-Komplexe zu detektieren. Ventrikuläre Depolarisationen kennzeichnen dabei ventrikuläre Eigenaktionen , d.h. intrinsische (natürliche) ventrikuläre Ereignisse. Ein derartiger implantierbarer Herzstimulator kann rechtsventrikuläre Ereignisse unabhängig von der rechtsventrikulären Sensingelektrode detektieren und erlaubt es, die mittels der rechtsventrikulären Sensingelektrode detektierten ventrikulären Ereignisse einem Plausibilitätstest zu unterziehen.

In Verbindung mit der letztgenannten Ausführungsvariante der zusätzlichen Erfassungseinrichtung ist die Auswerteeinheit vorzugsweise ausgebildet, einen Rhythmus dann als Normalrhythmus zu bewerten, wenn im Fernfeldelektrokardiogramm QRS-Komplexe sicher nachgewiesen werden können.

Vorzugsweise ist die zusätzliche Erfassungseinrichtung bzw. deren Fernfeldelektrokardiogramm-Auswerteeinheit ausgebildet, einen QRS-Komplexes im Fernfeldelektrokardiogramm mittels eines Mustervergleiches zu detektieren.

Alternativ kann die zusätzliche Erfassungseinrichtung bzw. deren Fernfeldelektrokardiogramm-Auswerteeinheit dazu ausgebildet sein, in dem Fernfeldelektrokardiogramm Senseereignisse durch Vergleich mit einem fest vorgegebenen oder einem variablen Schwellwert zu detektieren.

Gemäß weiterer Ausführungsvarianten des Herzstimulators kann dieser einen Defibrillationselektrodenleitungsanschluss besitzen, der für den Anschluss einer ventrikulären Defibrillationselektrodenleitung geeignet ist, die zwei Schockwendeln aufweist, nämlich eine proximale (beispielsweise zur Anordnung in der Vena cava superior vorgesehene) Schockwendel und eine distale Schockwendel. Dabei kann der Herzstimulator so ausgebildet sein, dass die beiden Schockwendeln unabhängig voneinander mit der Fernfeldelektrokardiogramm-Erfassungseinheit der zusätzlichen Erfassungseinrichtung zu verbinden sind. Vorzugsweise ist diese dazu ausgebildet, von den beiden zur Verfügung stehenden Schockwendeln mit der proximalen Schockwendel verbunden zu sein, um das Fernfeldelektrokardiogramm aus der Potenzialdifferenz zwischen proximaler Schockwendel und elektrisch leitender Oberfläche des Herzstimulatorgehäuses zu bestimmen.

Gemäß einer alternativen Ausführungsvariante kann der Herzstimulator auch so ausgebildet sein, dass die Fernfeldelektrokardiogramm-Erfassungseinheit der zusätzlichen Erfassungseinheit wahlweise mit einer proximalen oder einer distalen Schockwendel einer ventrikulären Defibrillationselektrodenleitung zu verbinden ist.

Geeignete Varianten des Anschlusses der zusätzlichen Erfassungseinrichtung an verschiede Elektroden zum Zwecke der Aufnahme eines Fernfeldelektrokardiogramms sind:
- die zusätzliche Erfassungseinrichtung ist zur Ableitung des Fernfeldelektrokardiogramms mit einer distalen Schockelektrode und dem Gehäuse des ICD verbunden, um das Fernfeldelektrokardiogramm zwischen diesen Elektroden abzuleiten.
- die zusätzliche Erfassungseinrichtung ist zur Ableitung des Fernfeldelektrokardiogramms mit einer proximalen Schockelektrode und dem Gehäuse des ICD verbunden, um das Fernfeldelektrokardiogramm zwischen diesen Elektroden abzuleiten.
- die zusätzliche Erfassungseinrichtung ist zur Ableitung des Fernfeldelektrokardiogramms mit einer distalen und einer proximalen Schockelektrode des ICD verbunden, um das Fernfeldelektrokardiogramm zwischen diesen Elektroden abzuleiten.
- die zusätzliche Erfassungseinrichtung ist zur Ableitung des Fernfeldelektrokardiogramms mit einer atrialen Sensing- oder Stimulationselektrode und dem Gehäuse des ICD verbunden, um das Fernfeldelektrokardiogramm zwischen diesen Elektroden abzuleiten.

Vorzugsweise ist der Herzstimulator ausgebildet, die Information über einen zurückgehaltenen Defibrillationsschock im Implantat zu speichern, so dass diese von einem Programmiergerät ausgelesen und angezeigt werden kann.

Weiter bevorzugt ist der Herzstimulator ausgebildet, die Information über einen zurückgehaltenen Defibrillationsschock im Implantat zu speichern und automatisch über eine drahtlose Datenübertragungsschnittstelle an ein Telemonitoring-System übertragen.

Gemäß einer weiteren, bevorzugten Ausführungsform besitzt der Herzstimulator einen Simulationsmodus für klinische Untersuchungen, in dem der Herzstimulator nur diagnostische Informationen im zuvor beschriebenen Sinne liefert, ohne jedoch tatsächlich Schocks zu inhibieren.

Außerdem weist der Herzstimulator vorzugsweise eine VT/SVT-Bewertungseinheit auf, die ausgebildet ist, anhand von aufeinander folgenden erfassten atrialen Ereignissen und aufeinander folgenden ventrikulären Ereignissen eine Zuordnung einer ventrikulären Tachyarrhythmie zu einer supraventrikulären Tachykardie (SVT) oder einer ventrikulären Tachykardie (VT) durchzuführen.

Weitere vorteilhafte Ausgestaltungen eines erfindungsgemäßen Herzstimulators ergeben sich aus hier nicht explizit erwähnten Kombinationen der verschiedenen vorteilhaften Merkmale eines solchen Herzschrittmachers, wie sie hier beschrieben sind.

Die Erfindung soll nun anhand eines Ausführungsbeispiels mit Bezug auf die Figuren näher erläutert werden. Von diesen zeigt:
- Fig. 1:: einen Herzstimulator in Form eines implantierbaren 3-Kammer ICD-Systems in Verbindung mit daran angeschlossenen Elektrodenleitungen;
- Fig. 2:: ein schematisches Blockschaltbild des Herzstimulators aus Figur 1;
- Fig. 3:: ein Blockschaltbild einiger relevanter Komponenten eines 3-Kammer ICD-System;
- Fig. 4:: einen Herzstimulator in Form eines implantierbaren 1-Kammer ICD-Systems;
- Fig. 5:: ein Blockschaltbild einiger relevanter Komponenten eines 1-Kammer ICD-Systems;
- Fig. 6:: ein Ablaufdiagramm für die Elektrodenfehlerdetektion in einem 3-Kammer-ICD;
- Fig. 7:: ein Beispiel für einen inadäquater Schock infolge Elektrodendefekts; und
- Fig. 8:: atriale und linksventrikuläre Marker aus Figur 7 in Verbindung mit den zu prüfenden Kriterien für die Elektrodenfehlererkennung.

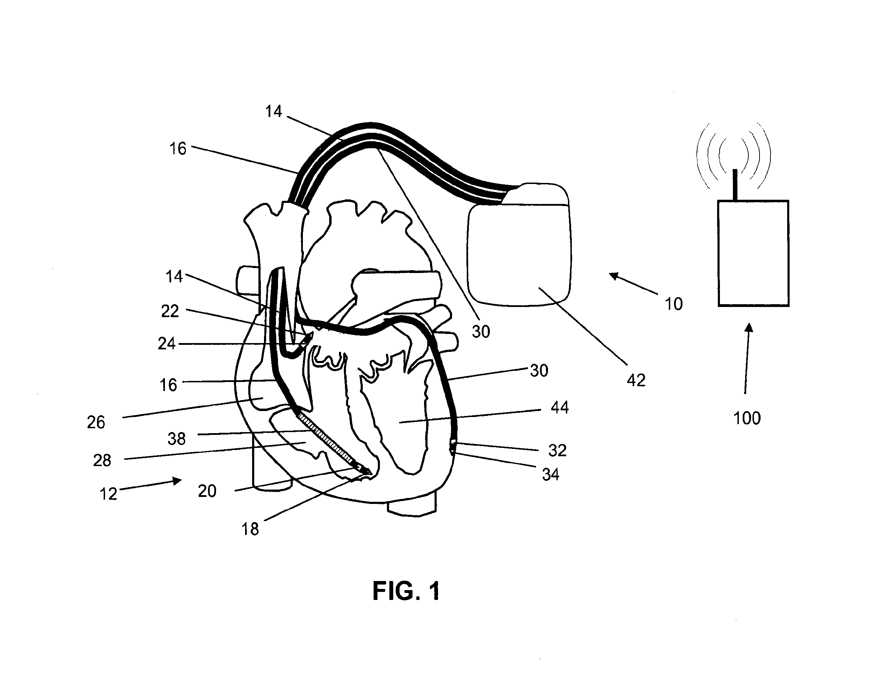

Fig. 1 zeigt ein Implantat 10 in Form eines biventrikulären Dreikammer-Herzschrittmachers und Cardioverters/Defibrillators (ICD). An diesen sind drei Elektrodenleitungen angeschlossen, nämlich eine rechtsatriale Elektrodenleitung 14, eine rechtsventrikuläre Elektrodenleitung 16 und eine linksventrikuläre Elektrodenleitung 30. Im implantierten Zustand endet die rechtsatriale Elektrodenleitung 14 im rechten Atrium 26 eines Herzens 12. Die rechtsventrikuläre Elektrodenleitung 16 endet im rechten Ventrikel 28 des Herzens 12 und die linksventrikuläre Elektrodenleitung 30 reicht über den Coronarsinus des Herzens 12 bis zum linken Ventrikel 44 des Herzens.

Die rechtsatriale Elektrodenleitung 14 trägt an ihrem distalen Ende eine rechtsatriale Spitzenelektrode RA Tip 22 und in geringem Abstand davon eine rechtsatriale Ringelektrode RA Ring 24. In ähnlicher Weise trägt die rechtsventrikuläre Elektrodenleitung 16 an ihrem distalen Ende eine rechtsventrikuläre Spitzenelektrode RV Tip 18 und wenig entfernt davon eine rechtsventrikuläre Ringelektrode RV Ring 20. Auch am distalen Ende der linksventrikulären Elektrodenleitung 30 ist eine linksventrikuläre Spitzenelektrode LV Tip 34 und wenig entfernt davon eine linksventrikuläre Ringelektrode LV Ring 32 angebracht. Diese Elektroden dienen der Aufnahme elektrischer Potenziale in der jeweiligen Herzkammer sowie der Abgabe von Stimulationsimpulsen an die jeweilige Herzkammer im normalen Schrittmacherbetrieb. Dieses braucht an dieser Stelle nicht mehr erläutert zu werden.

Die rechtsventrikuläre Elektrodenleitung 16 trägt außerdem noch eine im implantierten Zustand im rechten Ventrikel angeordnete rechtsventrikuläre Schockwendel 38 als Defibrillationselektrode.

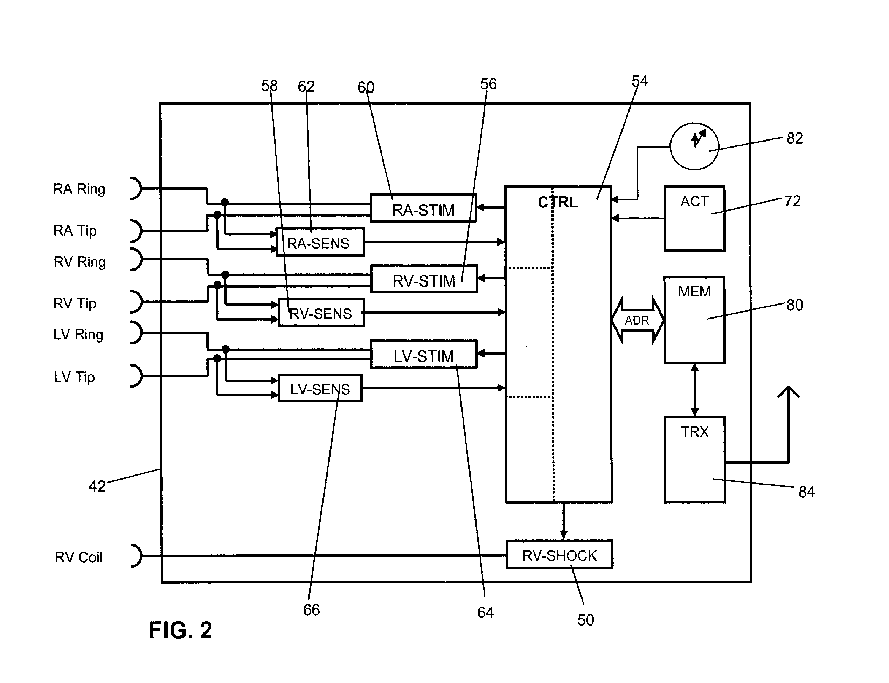

In Figur 2 sind die Hauptbestandteile des Herzstimulators 10 dargestellt. Auf der linken Seite sind die elektrischen Anschlüsse für die verschiedenen Elektroden 18, 20, 24, 22, 32, 34 und 38 dargestellt. Die Schockelektrode 38 ist mit einem rechtsventrikulären Schockimpulsgenerator 50 verbunden. Der Schockgenerator 50 ist mit einer Stimulationssteuereinheit 54 verbunden, die den Schockimpulsgenerator 50 bei Bedarf zur Erzeugung und Abgabe eines Defibrillationsschocks ansteuert.

Der Anschluss für die rechtsventrikuläre Spitzenelektrode RV Tip 18 sowie der Anschluss für die rechtsventrikuläre Ringelektrode RV Ring 20 sind jeweils sowohl mit einer rechtsventrikulären Stimulationseinheit 56 als auch mit einer rechtsventrikulären Sensingeinheit 58 verbunden. Sowohl die rechtsventrikuläre Stimulationseinheit 56 als auch die rechtsventrikuläre Sensingeinheit 58 sind jeweils mit der Stimulationssteuereinheit 54 verbunden.

Die rechtsventrikuläre Stimulationseinheit 56 ist dazu ausgebildet, auf ein Ansteuersignal der Stimulationssteuereinheit 54 hin einen rechtsventrikulären Stimulationsimpuls zu erzeugen und im Anschluss an die rechtsventrikuläre Ringelektrode RV Ring 20 und die rechtsventrikuläre Spitzenelektrode RV Tip 18 abzugeben. Alternativ ist es auch möglich, dass das Gehäuse 42 des Herzstimulators 10 eine neutrale Elektrode bildet und die rechtsventrikuläre Stimulationseinheit 56 mit dem Anschluss für die rechtsventrikuläre Spitzenelektrode RV Tip 18 und dem Gehäuse 42 als andere Elektrode zur Abgabe eines Stimulationsimpulses verbunden ist. Ein rechtsventrikulärer Stimulationsimpuls unterscheidet sich von einem Defibrillationsschock dadurch, dass der Stimulationsimpuls eine wesentlich geringere Impulsstärke besitzt, so dass er nicht wie ein Defibrillationsschock auf einen Schlag das vollständige Herzgewebe (Myokard) einer Herzkammer erregt, sondern nur die Herzmuskelzellen in unmittelbarer Umgebung der rechtsventrikulären Spitzenelektrode RV Tip 18. Diese Erregung breitet sich dann durch natürliche Reizleitung über den gesamten rechten Ventrikel 28 weiter aus und sorgt so für eine stimulierte Kontraktion des rechten Ventrikels 28.

Die rechtsventrikuläre Sensingeinheit 58 ist dazu ausgebildet, an dem Anschluss für die rechtsventrikuläre Ringelektrode RV Ring 20 und die rechtsventrikuläre Spitzenelektrode RV Tip 18 anliegende elektrische Potentiale zunächst durch einen Eingangsverstärker zu verstärken und zu filtern. Weiterhin ist die rechtsventrikuläre Sensingeinheit 58 ausgebildet, den Verlauf der an ihren Eingängen anliegenden elektrischen Signale derart auszuwerten, dass die rechtsventrikuläre Sensingeinheit 58 selbsttätig eine natürliche (intrinsische), d.h. selbsttätige Kontraktion des rechten Ventrikels 28 detektiert. Dies kann beispielsweise dadurch geschehen, dass der Verlauf des an den Eingängen der rechtsventrikulären Sensingeinheit 58 anliegenden Signals mit einem Schwellwert verglichen wird. Typischerweise ist die größte Amplitude des Signals in Form der sogenannten R-Zacke kennzeichnend für eine natürliche Kontraktion des rechten Ventrikels 28, die durch Schwellwertvergleich detektiert werden kann. Die rechtsventrikuläre Sensingeinheit 58 gibt daraufhin ein entsprechendes, eine natürliche Kontraktion des rechten Ventrikels 28 anzeigendes Ausgangssignal an die Stimulationssteuereinheit 54 aus.

In analoger Weise sind der Anschluss für die rechtsatriale Spitzenelektrode RA Tip 22 und der Anschluss für die rechtsatriale Ringelektrode RA Ring 24 sowohl mit einer rechtsatrialen Stimulationseinheit 60 als auch mit einer rechtsatrialen Sensingeinheit 62 verbunden, die jeweils ihrerseits wiederum mit der Stimulationssteuereinheit 54 verbunden sind. Die rechtsatriale Stimulationseinheit 60 ist dazu ausgebildet, Stimulationsimpulse zu erzeugen, deren Stärke ausreicht, das rechtsatriale Myokard zu erregen. Die rechtsatrialen Stimulationsimpulse können dabei eine andere Impulsstärke besitzen, als die rechtsventrikulären Stimulationsimpulse. Die rechtsatriale Sensingeinheit 62 ist ausgebildet, aus dem Verlauf des an ihren Eingängen anliegenden Differenzsignals eine sogenannte P-Welle zu detektieren, die eine natürliche (intrinsische) Kontraktion des rechten Atriums 26 kennzeichnet. Detektiert die rechtsatriale Sensingeinheit 62 eine entsprechende P-Welle, erzeugt sie ein Ausgangssignal und gibt dieses an die Stimulationssteuereinheit 54 weiter, welches eine natürliche Kontraktion des rechten Atriums 26 kennzeichnet.

In gleicher Weise sind auch der Anschluss für die linksventrikuläre Spitzenelektrode LV Tip 34 und der Anschluss für die linksventrikuläre Ringelektrode LV Ring 32 mit einer linksventrikulären Stimulationseinheit 64 und einer linksventrikulären Sensingeinheit 66 verbunden. Die linksventrikuläre Stimulationseinheit 64 und die linksventrikuläre Sensingeinheit 66 sind ebenso mit der Stimulationssteuereinheit 54 verbunden. Beide funktionieren ähnlich wie die bereits beschriebenen Stimulationseinheiten 56 und 60 und Sensingeinheiten 58 und 62.

Als weiterer Bestandteil des Herzstimulators 10 ist ein Aktivitätssensor 72 mit der Stimulationssteuereinheit 54 verbunden und in das Gehäuse 42 des Herzstimulators 10 integriert. Der Aktivitätssensor 72 ist dazu ausgebildet, ein von der körperlichen Aktivität eines Patienten abhängiges Bewegungssignal zu erfassen und ein entsprechendes, die körperliche Aktivität des Patienten anzeigendes Signal an die Stimulationssteuereinheit 54 auszugeben. Dies erlaubt es, dass die Stimulationssteuereinheit 54 das Timing der Stimulationsimpulse an den Bedarf des Patienten (hämodynamischen Bedarf) anpasst.

Weiterhin umfasst der Herzstimulator 10 eine Speichereinheit 80, die mit der Stimulationssteuereinheit 54 verbunden ist und es erlaubt, von der Stimulationssteuereinheit 54 erzeugte oder ausgewertete Signale zu speichern. Andererseits erlaubt es die Speichereinheit 80, Steuerprogramme für die Stimulationssteuereinheit 54 in veränderbarer Form zu speichern. Des Weiteren ist die Stimulationssteuereinheit 54 mit einem Zeitgeber 82 verbunden.

Die Speichereinheit 80 ist mit einer Telemetrieeinheit 84 verbunden, die es erlaubt, in der Speichereinheit 80 abgelegte Daten drahtlos an das externe Gerät 100 zu übertragen oder Programmierbefehle seitens des externen Geräts 100 zu dem Herzstimulator 10 zu übertragen und in der Speichereinheit 80 zu speichern.

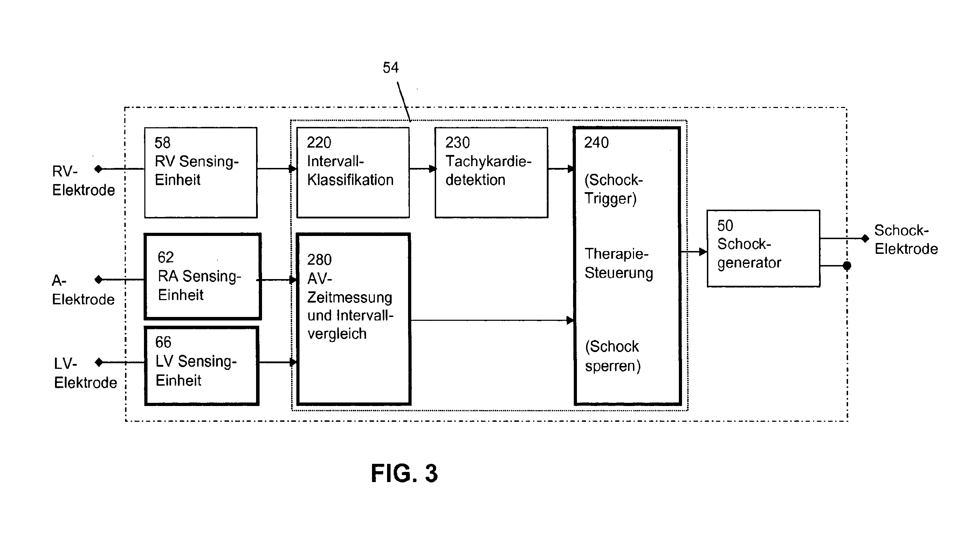

Die Steuereinheit 54 ist dazu ausgebildet, eine primäre Kammerflimmerdetektion (VF-Detektion) auf Basis des rechtsventrikulären, bipolar abgeleiteten und von der rechtsventrikulären Sensingeinheit aufgenommenen IEGMs auszuführen. Dazu weist die Steuereinheit 54 eine Intervallklassifikationseinheit 220 (siehe Figur 3) auf. Ist eine vorgegebene Detektionsbedingung (X aus Y-Kriterium) erfüllt, überprüft die Steuereinheit 54 mittels einer zusätzlichen Erfassungseinrichtung und einer Auswerteinheit 280 (siehe Figur 3), ob die Ereignisfolgen, aufgezeichnet über die atriale Elektrode 14 und linksventrikuläre Elektrode 30 eine definierte Zeitbedingung erfüllen, die einem Kammerflimmern widersprechen. In diesem Falle wird die Defibrillationsschockabgabe inhibiert, andernfalls der Schock regulär abgegeben. Geprüft wird dabei das Auftreten eines linksventrikulären Ereignisses (Sense) in einem definierten Zeitfenster nach atrialer Stimulation oder Wahrnehmung und ein Vergleich der atrialen mit den linksventrikulären Intervalllängen.

Fig. 3 zeigt einige der für die vorliegende Erfindung relevanten Komponenten des Herzstimulators 10 und insbesondere der Steuereinheit 54. Wie beschrieben, besitzt der Herzstimulator 10 Anschlüsse für eine rechtsventrikuläre (RV) Elektrodenleitung 16, eine atriale (A) Elektrodenleitung 14 und linksventrikuläre (LV) Elektrodenleitung 30 mit entsprechenden Stimulations- und Wahrnehmungselektroden. Defibrillationsschocks können über die Defibrillationsschockelektrode 38 und das Gehäuse 42 als Gegenelektrode für die Schockabgabe abgegeben werden.

Die rechtsventrikuläre Elektrodenleitung 16 ist verbunden mit der rechtsventrikulären Sensingeinheit 58. Diese Sensingeinheit 58 verstärkt, digitalisiert und filtert zunächst das mittels der Sensingelektroden 18 und 20 abgeleitete IEGM und extrahiert daraus die Zeitpunkte der rechtsventrikulären Depolarisation (Sense). Diese Sense-Ereignisse werden dann von einem Intervallklassifikator 220 als Bestandteil der Steuereinheit 54 nach der Intervalllänge bewertet und anschließend an einem Detektionszähler 230 als weiterem Bestandteil der Steuereinheit 54 für die VF-Detektion zugeführt. Dieser VF-Detektionszähler 230 ist vorzugsweise ein X aus Y - Zähler, d.h. es müssen für ein erfülltes VF-Detektionskriterium X aus zurückliegenden Y Intervallen die VF-Zonengrenze überschreiten. Wird dieses VF-Kriterium erfüllt, dann wird ein Triggersignal an die Therapiesteuereinheit 240 als Bestandteil der Steuereinheit 54 zur Einleitung einer Schocktherapie mit den programmierten Parametern gesendet. Diese Therapiesteuerung 240 veranlasst dann das Aufladen der Hochspannungskondensatoren und die anschließende Schockabgabe mittels Schockgenerator 50.

Die atriale Elektrodenleitung 14 und die linksventrikuläre Elektrodenleitung 30 sind ebenfalls mit jeweils einer Sensingstufe (62, 66) verbunden, um die atrialen bzw. linksventrikulären Depolarisationen zu registrieren. Diese werden dann einer Messeinheit 280 als Bestandteil der Steuereinheit 54 zugeführt, die sowohl die A-LV-Zeit, als auch die atrialen (PP) und linksventrikulären (LV) Intervalle bestimmt. Die Messeinheit 280 führt weiterhin eine Prüfung der A-LV-Zeiten und einen Vergleich der PP- und LV-Intervalle zuzüglich Toleranz durch. Die Vergleichsparameter (A-LV-Zeiterwartung, Toleranz des Intervallvergleichs, Zählerkriterien, minimal zulässiges LV-Intervall) können dabei durch eine Programmierung von außen beeinflusst werden. Sind die entsprechenden Kriterien in der Messeinheit 280 erfüllt, generiert die Messeinheit 280 ein Signal zum Sperren der Schocktherapie an die Therapiesteuereinheit 240 weitergereicht. Ist in dieser Therapiesteuereinheit der Parameter "Schocktherapie bei Elektrodenfehler sperren" aktiviert, so setzt sich das Signal zum Sperren der Schocktherapie gegenüber dem Schocktrigger durch und inhibiert so die Schockabgabe im Falle eines rechtsventrikulären Elektrodenfehlers. Die Messeinheit 280 fungiert in dieser Konfiguration in Verbindung mit der atrialen und der linksventrikulären Sensingeinheit 62 bzw. 66 als zusätzliche Erfassungseinheit im Sinne der Erfindung.

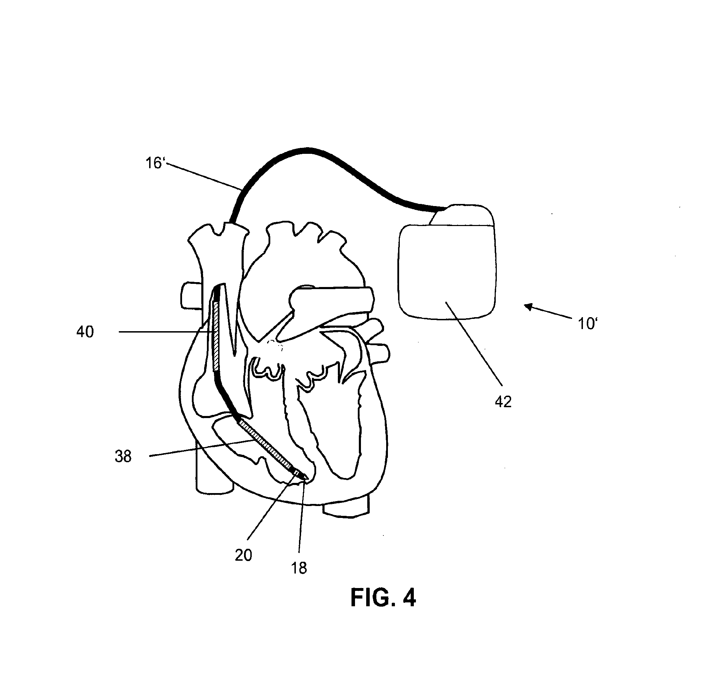

In Figur 4 ist ein Herzstimulator 10' in Form eines 1-Kammer-ICDs dargestellt. Der Herzstimulator 10' ist dabei mit einer rechtsventrikulären Elektrodenleitung 16' verbunden, die neben den Elektroden 18 und 20 für die Stimulation und das Sensing auch zwei Schock-Elektroden, nämlich eine distale Schockelektrode 38 und eine proximale Schockelektrode 40 trägt.

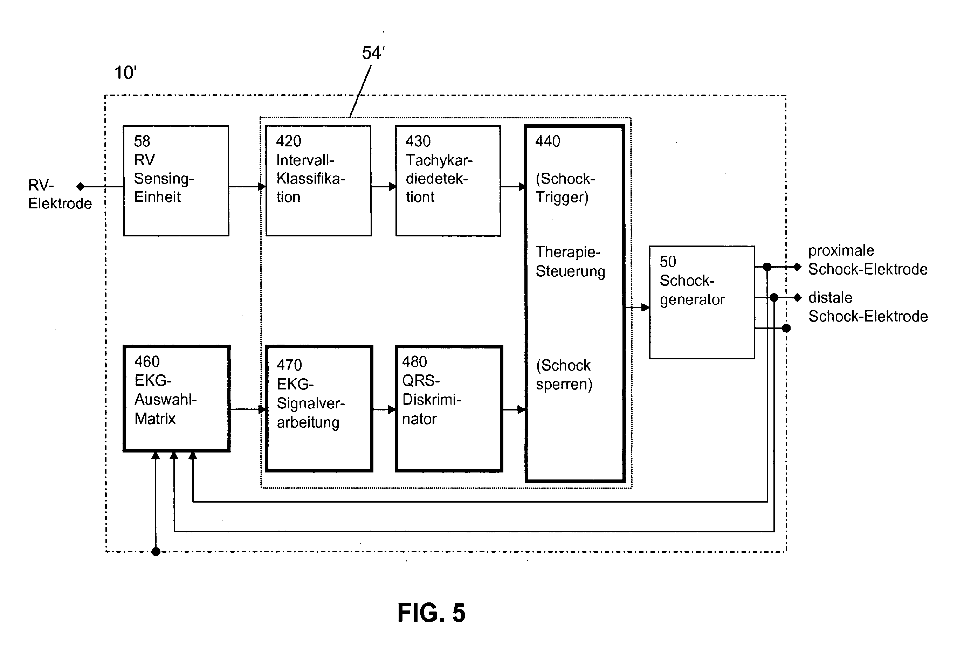

Das Blockschaltbild für das 1-Kammer-ICD-System 10' ist auszugsweise in Figur 5 dargestellt. Der Herzstimulator 10' besitzt Anschlüsse für eine rechtsventrikuläre Elektrodenleitung 16' mit Stimulations- und Wahrnehmungselektroden 18 und 20 und besitzt Anschlüsse für zwei Defibrillationsschockelektroden 38 und 40, die beide auf der rechtsventrikulären Elektrodenleitung 16' angeordnet sind. Das wenigstens teilweise elektrisch leitende Gehäuse 42 des ICD 10' stellt eine weitere Schockelektrode für die Schockabgabe dar. Der für die Defibrillation genutzte Schockpfad ist dabei über eine Auswahlmatrix, beispielsweise die Auswahlmatrix 460 einstellbar.

Die rechtsventrikuläre Elektrodenleitung 16' ist verbunden mit der (einzigen) rechtsventrikulären Sensingeinheit 58'. Diese Sensingeinheit 58' verstärkt, digitalisiert und filtert zunächst das mittels der Elektrodenleitung 16' abgeleitete IEGM und extrahiert daraus die Zeitpunkte der rechtsventrikulären Depolarisation (Sense). Diese Sense-Ereignisse werden dann von einem Intervallklassifikator 420 als Bestandteil der Steuereinheit 54' nach der Intervalllänge bewertet und anschließend an einen Detektionszähler 430 als weiterem Bestandteil der Steuereinheit 54' für die VF-Detektion (primäre Kammerflimmerdetektion) zugeführt. Dieser VF-Detektionszähler ist vorzugsweise ein X aus Y - Zähler, d.h. es müssen für ein erfülltes VF-Detektionskriterium X aus zurückliegenden Y Intervallen die VF-Zonengrenze überschreiten. Wird dieses VF-Kriterium erfüllt, dann wird ein Triggersignal an die Therapiesteuereinheit 440 als Bestandteil der Steuereinheit 54' zur Einleitung einer Schocktherapie mit den programmierten Parametern gesendet. Diese Therapiesteuerung 440 veranlasst dann das Aufladen der Hochspannungskondensatoren und die anschließende Schockabgabe mittels Schockgenerator 50.

Die Steuereinheit 54' weist eine zusätzliche Erfassungseinrichtung auf, die eine EKG-Auswahlmatrix 460 und eine EKG-Signalverarbeitungseinheit 470 umfasst und zur Aufnahme eines Fernfeldelektrokardiogramms dient. Mit diesen verbunden ist eine Auswerteeinheit 480, die dazu ausgebildet ist, zu ermitteln ob ein mittels der zusätzlichen Erfassungseinrichtung 460 und 470 aufgenommenes Fernfeldelektrogramm QRS Komplexe enthält, deren Morphologie einem Kammerflimmern widerspricht.

Die Anschlüsse für die Schockelektroden 38 und 40 und das Gehäuse 42 des ICD 10' sind elektrisch an die EKG-Auswahlmatrix 460 angeschlossen. Mit dieser Auswahlmatrix 460 kann durch Programmierung eine der folgenden Elektrodenkonfigurationen für die Ableitungen für eines Fernfeldelektrokardiogramms eingestellt werden:
- distale Schockelektrode gegen Gehäuse (38 - 42)
- proximale Schockelektrode gegen Gehäuse (40 - 42)
- distale gegen proximale Schockelektrode (38 - 40).

Die an den ausgewählten Elektroden anliegenden Potentialdifferenzen werden anschließend in der EKG-Signalverarbeitungseinheit 470 verstärkt, digitalisiert und gefiltert und so ein Fernfeldelektrokardiogramm gewonnen. Dieses vorverarbeitete Signal wird dann einem QRS-Diskriminator 480 zugeführt, um das Vorhandensein von QRS-Komplexen im Signal zu bestimmen. Der QRS-Diskriminator ist dabei vorzugsweise als digitaler Signalprozessor (DSP) ausgeführt, um universelle Algorithmen zur Signalverarbeitung implementieren zu können. Der QRS-Diskriminator prüft, ob in den zurückliegenden 2...n Sekunden ein QRS-Komplex im Far-Field-IEGM vorhanden ist. Ist dieses Kriterium in dem als Auswerteeinheit 480 dienenden QRS-Diskriminator erfüllt, so wird ein Signal zum Sperren der Schocktherapie an die Therapiesteuereinheit 440 weitergereicht. Ist in dieser Therapiesteuereinheit der Parameter "Schocktherapie bei Elektrodenfehler sperren" aktiviert, so setzt sich das Signal zum Sperren der Schocktherapie gegenüber dem Schocktrigger durch und inhibiert so die Schockabgabe im Falle eines rechtsventrikulären Elektrodenfehlers.

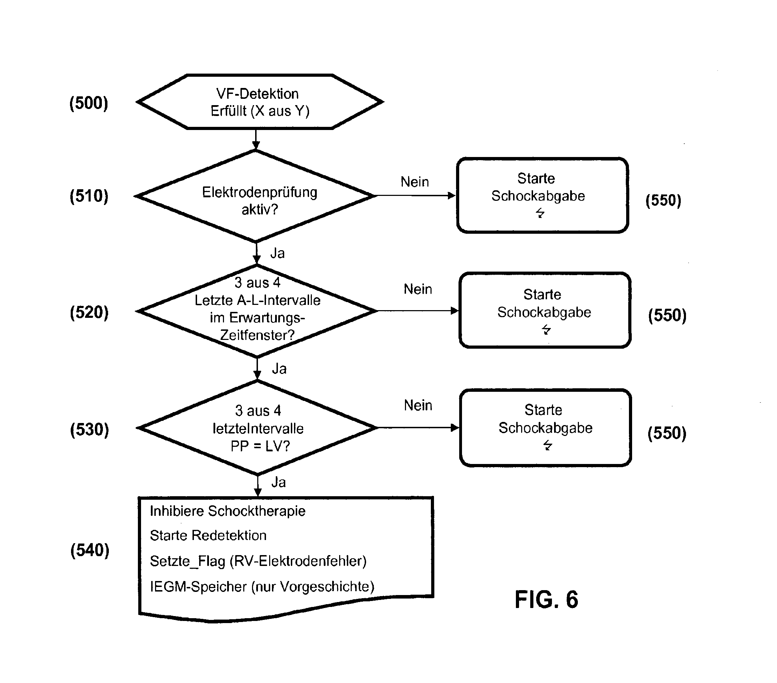

Figur 6 zeigt das Ablaufdiagramm, das die Steuereinheit 54 hinsichtlich ihrer Funktionsweise bei der Elektrodenfehlerdetektion in dem 3-Kammer-ICD 10 aus Figuren 1 bis 3 beschreibt. Nach erfülltem VF-Detektionskriterium 500, basierend auf rechtsventrikulärem Sensing, wird geprüft, ob die Funktion zur Elektrodenprüfung aktiviert ist 510. Ist diese Funktion inaktiv, dann wird der Defibrillationsschock regulär abgegeben 550. Ist die Funktion eingeschaltet, dann prüft das Implantat, ob in den zurückliegenden 4 A-LV-Ereignisfolgen mindestens 3 mal ein LV-Sense innerhalb des programmierten Erwartungsintervalls nach einem atrialen Ereignis liegt. Das 3 aus 4 Kriterium macht den Algorithmus dabei robust gegenüber Extrasystolen. Ist diese Bedingung nicht erfüllt, dann wird regulär der Schock abgegeben 550, andernfalls erfolgt eine weitere Prüfung, ob die zurückliegenden atrialen Intervalle (PP) den LV-Intervallen entsprechen 530 und die LV-Intervalle nicht schneller als die programmierte VF-Zykluslänge sind. Diese Prüfung ist ebenfalls an ein 3 aus 4 Kriterium geknüpft und lässt zudem eine programmierbare Toleranz (z.B. +/- 24ms) zu. Sind die Intervalle nicht identisch, dann wird regulär der Schock abgegeben 550. Sind die Intervalle übereinstimmend, dann wird die Schocktherapie inhibiert und anstelle dessen eine Redetektion gestartet, das Flag zur Anzeige eines rechtsventrikulären Elektrodenfehlers gesetzt und im IEGM-Speicher die sog. Vorgeschichte der VF-Detektion abgespeichert (z.B. die letzten 10 Sekunden vor erfüllter VF-Detektion). Ferner kann diese Information an ein Fernüberwachungssystem automatisch übertragen werden.

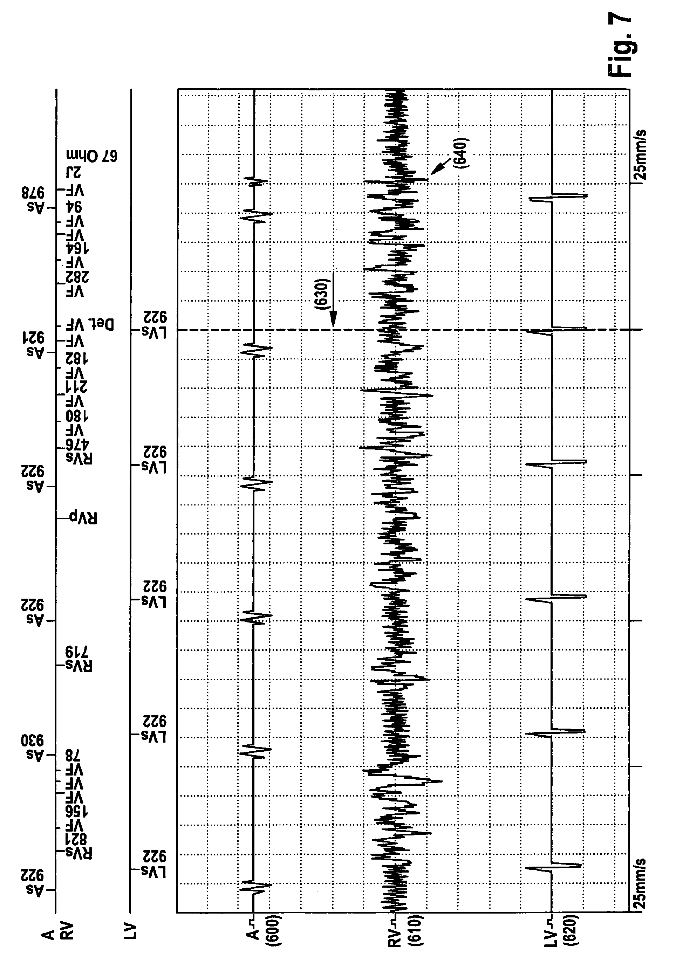

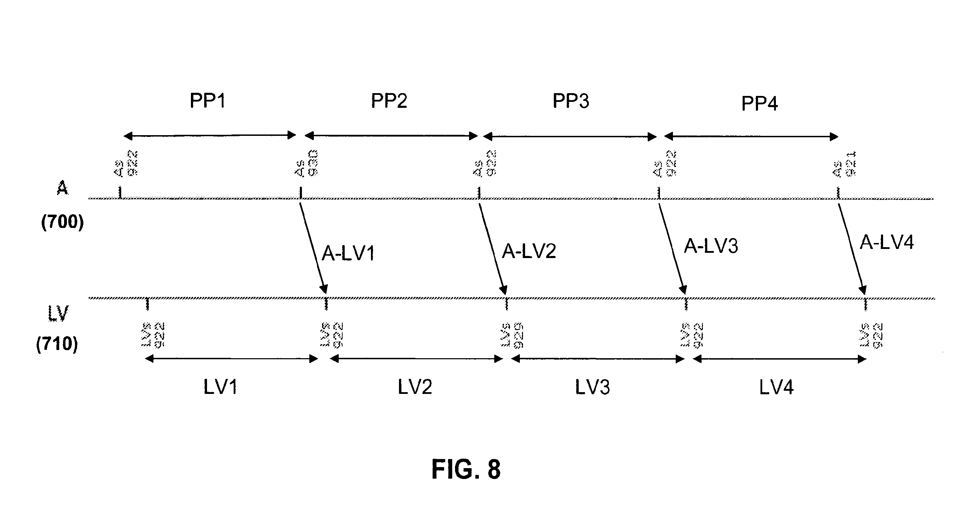

Figuren 7 und 8 illustrieren die Wirksamkeit dieser Betriebsweise zur Elektrodenfehlerdetektion. In Figur 7 ist ein typisches Beispiel einer inadäquaten Schockabgabe infolge einer defekten RV-Elektrode 610 dargestellt. In den IEGM-Kanälen sind das atriale IEGM 600, das gestörte rechtsventrikuläre IEGM 610 und das linksventrikuläre IEGM 620 sichtbar. Aufgrund der Störungen im RV-Kanal 610 wird das Kriterium zur VF-Detektion (hier 8 aus 12) erfüllt 630 und daraufhin ein Schock 640 an den Patienten abgegeben.

In Figur 8 sind die atrialen 700 und linksventrikulären Marker 710 von Figur 7 dargestellt. Zusätzlich sind die im beschriebenen Verfahren zu prüfenden Kriterien für die Elektrodenfehlererkennung gezeichnet. Die Ablaufsteuerung prüft zunächst die A-LV-Intervalle für die zurückliegenden 4 A-LV-Folgen (A-LV1...4). In diesem Beispiel liegen diese innerhalb des definierten Erwartungsfensters. Ebenso stimmen die PP und LV-Intervalle überein (zulässige Toleranz +/-24ms: PP1=LV1; PP2=LV2; PP3=LV3; PP4=LV4). Die Ablaufsteuerung würde in diesem Fall die RV-Elektrode als defekt deklarieren und die Schockabgabe inhibieren.

## Patentansprüche

1. Implantierbarer Dreikammer-Herzstimulator (10), mit
- einem wenigstens zum Teil elektrisch leitenden Gehäuse (42), und
- einem Defibrillationselektrodenleitungsanschluss für den Anschluss einer ventrikulären Defibrillationselektrode (38, 40) mittels einer Elektrodenleitung (16),
- einem Defibrillationsschockgenerator (50), der mit dem Defibrillationselektrodenleitungsanschluss verbunden und ausgebildet ist, einen Defibrillationsschock zu generieren und über den Defibrillationselektrodenleitungsanschluss abzugeben
- einer rechtsventrikulären Sensingeinheit (58), die mit wenigstens einer rechtsventrikulären, gegenüber der Defibrillationselektrode (38, 40) relativ kleinflächigen Sensingelektrode (18, 20) über eine rechtsventrikuläre Elektrodenleitung (16, 16') verbunden oder zu verbinden und ausgebildet ist, elektrische Potentiale des ventrikulären Myokards aufzunehmen und zu verarbeiten,
- einer linksventrikulären Sensingeinheit (66), die mit wenigstens einer linksventrikulären Sensingelektrode (32, 34) über eine linksventrikuläre Elektrodenleitung (16) verbunden oder zu verbinden und ausgebildet ist, elektrische Potentiale des ventrikulären Myokards aufzunehmen und zu verarbeiten,
- einer rechtsatrialen Sensingeinheit (62), die mit wenigstens einer rechtsatrialen Sensingelektrode (22, 24) über eine rechtsatriale Elektrodenleitung (14) verbunden oder zu verbinden und ausgebildet ist, elektrische Potentiale des atrialen Myokards aufzunehmen und zu verarbeiten,
- einer Steuereinheit (54; 54'), die mit der rechtsventrikulären Sensingeinheit (58) und dem Defibrillationsschockgenerator (50) verbunden und die ausgebildet ist, ventrikuläres Kammerflimmern zu detektieren und je nach Detek-tionsergebnis den Defibrillationsschockgenerator (50) zur Abgabe eines Defibrillationsschocks anzusteuern,
- einer zusätzlichen Erfassungseinrichtung (280; 460, 470) zur Wahrnehmung ventrikulärer und atrialer Ereignisse, die unabhängig von der wenigstens einen rechtsventrikulären Sensingelektrode arbeitet,
wobei die Steuereinheit (54; 54') mit der zusätzlichen Erfassungseinrichtung (280; 460, 470) verbunden und ausgebildet ist, bei sicherem Nachweis eines Normalrhythmus mit der zusätzlichen Erfassungseinrichtung (280; 460, 470) die Abgabe eines Defibrillationsschocks zu inhibieren,
**dadurch gekennzeichnet,**
**dass** die Auswerteeinheit (280) ausgebildet ist, einen Rhythmus dann als Normalrhythmus zu bewerten, wenn nach einem intrinsischen oder stimulierten atrialen Ereignis innerhalb eines vorgegebenen Zeitfensters "AV-Zeiterwartung" ein einziges linksventrikuläres Ereignis wahrgenommen wird und außerdem das Intervall zwischen aufeinander folgenden linksventrikulären Ereignissen dem Intervall der vorausgegangenen atrialen Ereignisse zuzüglich einer Toleranz entspricht.

2. Implantierbarer Herzstimulator gemäß Anspruch 1, **dadurch gekennzeichnet, dass** das vorgegebene Zeitfenster "AV-Zeiterwartung" für die linksventrikuläre Wahrnehmung nach atrialen Ereignissen manuell einstellbar ist.

3. Implantierbarer Herzstimulator nach einem der Ansprüche 1 bis 2, **dadurch gekennzeichnet, dass** die zusätzliche Erfassungseinrichtung (460, 470) zur Ableitung eines Fernfeldelektrokardiogramms ausgebildet und mit zwei im Vergleich zu einem bipolaren Sensingelektrodenpaar relativ weit voneinander entfernten, vergleichsweise großflächigen Elektroden verbunden oder zu verbinden ist.

4. Implantierbarer Herzstimulator nach einem der Ansprüche 1 bis 3 mit einem wenigstens teilweise elektrisch leitendem Gehäuse (42), **dadurch gekennzeichnet, dass** die zusätzliche Erfassungseinrichtung
- eine Fernfeldelektrokardiogrammerfassungseinheit (460, 470) aufweist, die einen ersten Eingang aufweist, der mit einem Anschluss für eine ventrikuläre Defibrillationselektrode sowie einen zweiten Eingang, der mit dem elektrisch leitenden Gehäuse (42) verbunden ist, und die ausgebildet ist, auf Basis der an den beiden Eingängen im Betrieb anliegenden elektrischen Potentiale ein Fernfeldelektrokardiogramm zu generieren,
sowie
- eine Fernfeldelektrokardiogrammauswerteeinheit (480),
die mit der Fernfeldelektrokardiogrammerfassungseinheit (460, 470) verbunden und
die ausgebildet ist, in einem von der Fernfeldelektrokardiogrammerfassungseinheit (460, 470) generierten Fernfeldelektrokardiogramm für ventrikuläre Depolarisationen charakteristische Signalmerkmale zu detektieren.

5. Implantierbarer Herzstimulator gemäß Anspruch 4, **dadurch gekennzeichnet, dass** der Anschluss für eine ventrikuläre Defibrillationselektrode für den Anschluss einer ventrikulären Elektrodenleitung (16') mit zwei Schockwendeln, nämlich einer proximalen Schockwendel (40) und einer distalen Schockwendel (38), derart ausgebildet ist, dass die Schockwendeln unabhängig voneinander mit der Fernfeldelek-trokardiogrammerfassungseinheit (460, 470) zu verbinden sind.

6. Implantierbarer Herzstimulator gemäß Anspruch 1, **dadurch gekennzeichnet, dass** die zusätzliche Erfassungseinrichtung eine Fernfeldelektrokardiogrammerfassungseinheit (460, 470) aufweist,
die mit einer ventrikulären Elektrodenleitung (16') mit zwei Schockwendeln verbunden oder zu verbinden und ausgebildet ist, ein Fernfeldelektrokardiogramm zwischen den beiden Schockwendeln abzuleiten,
sowie
- eine Fernfeldelektrokardiogrammauswerteeinheit (480),
die mit der Fernfeldelektrokardiogrammerfassungseinheit (460, 470) verbunden und
die ausgebildet ist, in einem von der Fernfeldelektrokardiogrammerfassungseinheit (460, 470) generierten Fernfeldelektrokardiogramm für ventrikuläre Depolarisationen charakteristische Signalmerkmale zu detektieren.

7. Implantierbarer Herzstimulator nach einem der Ansprüche 1 bis 4 mit einem wenigstens teilweise elektrisch leitendem Gehäuse (42), **dadurch gekennzeichnet, dass** die zusätzliche Erfassungseinrichtung
- eine Fernfeldelektrokardiogrammerfassungseinheit (460, 470) aufweist,
die einerseits mit einer atrialen Elektrode und andererseits mit dem elektrisch leitenden Gehäuse verbunden oder zu verbinden und ausgebildet ist, ein Fernfeldelektrokardiogramm zwischen diesen beiden Elektroden abzuleiten,
sowie
- eine Fernfeldelektrokardiogrammauswerteeinheit (480),
die mit der Fernfeldelektrokardiogrammerfassungseinheit (460, 470) verbunden und
die ausgebildet ist, in einem von der Fernfeldelektrokardiogrammerfassungseinheit (460, 470) generierten Fernfeldelektrokardiogramm für ventrikuläre Depolarisationen charakteristische Signalmerkmale zu detektieren.

8. Implantierbarer Herzstimulator nach einem der Ansprüche 1 bis 7, **dadurch gekennzeichnet, dass** die Auswerteeinheit (480) vorzugsweise ausgebildet ist, einen Rhythmus dann als Normalrhythmus zu bewerten, wenn im Fernfeldelektrokardiogramm QRS-Komplexe sicher nachgewiesen werden können.

9. Implantierbarer Herzstimulator nach einem der Ansprüche 1 bis 8, **dadurch gekennzeichnet, dass** die zusätzliche Erfassungseinrichtung bzw. deren Fernfeldelektrokardiogramm-Auswerteeinheit (480) ausgebildet einen QRS-Komplexes im Fernfeldelektrokardiogramm mittels eines Mustervergleiches zu detektieren.

10. Implantierbarer Herzstimulator nach einem der Ansprüche 1 bis 9, **dadurch gekennzeichnet, dass** die Fernfeldelektrokardiogramm-Auswerteeinheit (480) ausgebildet ist, ein Fernfeldelektrokardiogramm einer Signalformanalyse zur Bestimmung von Signalspitzen zu unterziehen, die ventrikulären Ereignissen zuzuordnen sind.

11. Implantierbarer Herzstimulator nach einem der Ansprüche 1 bis 8, **dadurch gekennzeichnet, dass** die Fernfeldelektrokardiogramm-Auswerteeinheit (480) ausgebildet ist, in dem Fernfeldelektrokardiogramm Sense-Ereignisse durch Vergleich mit einem fest vorgegebenen oder variablen Schwellwert zu detektieren.

12. Implantierbarer Herzstimulator nach einem der Ansprüche 1 bis 11, **dadurch gekennzeichnet, dass** der implantierbare Herzstimulator einen Speicher (80) aufweist und ausgebildet ist, Information über einen zurückgehaltenen Defibrillationsschock in dem Speicher (80) zu speichern.

## Claims

1. An implantable triple-chamber cardiac stimulator (10), comprising
- an at least partly electrically conductive housing (42) and
- a defibrillation electrode line connector for the connection of a ventricular defibrillation electrode (38, 40) by means of an electrode line (16),
- a defibrillation shock generator (50) that is connected to the defibrillation electrode line connector and that is configured to generate a defibrillation shock and to deliver said shock via the defibrillation electrode line connector,
- a right-ventricular sensing unit (58) that is connected or is to be connected via a right-ventricular electrode line (16, 16') to at least one right-ventricular sensing electrode (18, 20) of relatively small surface area compared to the defibrillation electrode (38, 40) and that is configured to receive and to process electric potentials of the ventricular myocardium,
- a left-ventricular sensing unit (66) that is connected or is to be connected via a left-ventricular electrode line (16) to at least one left-ventricular sensing electrode (32, 34) and that is configured to receive and to process electric potentials of the ventricular myocardium,
- a right-atrial sensing unit (62) that is connected or is to be connected via a right-atrial electrode line (14) to at least one right-atrial sensing electrode (22, 24) and that is configured to receive and to process electric potentials of the atrial myocardium,
- a control unit (54; 54') that is connected to the right-ventricular sensing unit (58) and to the defibrillation shock generator (50) and that is configured to detect ventricular fibrillation and to actuate the defibrillation shock generator (50) to deliver a defibrillation shock depending on the detection result,
- an additional detection arrangement (280; 460, 470) for perceiving ventricular and atrial events that operates independently of the at least one right-ventricular sensing electrode,
wherein the control unit (54; 54') is connected to the additional detection arrangement (280; 460, 470) and is configured to inhibit the delivery of a defibrillation shock in the event that a normal rhythm is reliably detected with the additional detection arrangement (280; 460, 470),
**characterised in that**
the evaluation unit (280) is configured to then assess a rhythm as a normal rhythm if, following an intrinsic or stimulated atrial event, a single left-ventricular event is perceived within a predefined time window "AV time expectation" and if, in addition, the interval between successive left-ventricular events corresponds to the interval of the prior atrial events plus a tolerance.

2. The implantable cardiac stimulator according to Claim 1, **characterised in that** the predefined time window "AV time expectation" for the left-ventricular perception following atrial events can be manually set.

3. The implantable cardiac stimulator according to one of Claims 1 to 2, **characterised in that** the additional detection arrangement (460, 470) is configured to record a far-field electrocardiogram and is connected or is to be connected to two electrodes of comparatively large surface area that are distanced relatively far from one another compared to a bipolar sensing electrode pair.

4. The implantable cardiac stimulator according to one of Claims 1 to 3, comprising an at least partly electrically conductive housing (42), **characterised in that** the additional detection arrangement
- has a far-field electrocardiogram detection unit (460, 470)
that has a first input connected to a connector for a ventricular defibrillation electrode and also a second input connected to the electrically conductive housing (42), and
that is configured to generate a far-field electrocardiogram on the basis of the electric potentials applied to both inputs during operation,
and also has
- a far-field electrocardiogram evaluation unit (480)
that is connected to the far-field electrocardiogram detection unit (460, 470) and
that is configured to detect signal features characteristic for ventricular depolarisations in a far-field electrocardiogram generated by the far-field electrocardiogram detection unit (460, 470).

5. The implantable cardiac stimulator according to Claim 4, **characterised in that** the connector for a ventricular defibrillation electrode for the connection of a ventricular electrode line (16') having two shock coils, specifically a proximal shock coil (40) and a distal shock coil (38), is configured in such a way that the shock coils are to be connected independently of one another to the far-field electrocardiogram detection unit (460, 470).

6. The implantable cardiac stimulator according to Claim 1, **characterised in that** the additional detection arrangement has a far-field electrocardiogram detection unit (460,470)
that is connected or is to be connected to a ventricular electrode line (16') having two shock coils and is configured to record a far-field electrocardiogram between the two shock coils,
and also has
- a far-field electrocardiogram evaluation unit (480)
that is connected to the far-field electrocardiogram detection unit (460, 470) and
that is configured to detect signal features characteristic for ventricular depolarisations in a far-field electrocardiogram generated by the far-field electrocardiogram detection unit (460, 470).

7. The implantable cardiac stimulator according to one of Claims 1 to 4 comprising an at least partly electrically conductive housing (42), **characterised in that** the additional detection arrangement
- has a far-field electrocardiogram detection unit (460, 470) that is connected or is to be connected on the one hand to an atrial electrode and on the other hand to the electrically conductive housing and that is configured to record a far-field electrocardiogram between these two electrodes,
and also has
- a far-field electrocardiogram evaluation unit (480)
that is configured to be connected to the far-field electrocardiogram detection unit (460, 470) and
that is configured to detect signal features characteristic for ventricular depolarisations in a far-field electrocardiogram generated by the far-field electrocardiogram detection unit (460, 470).

8. The implantable cardiac stimulator according to one of Claims 1 to 7, **characterised in that** the evaluation unit (480) is preferably configured to assess a rhythm as a normal rhythm if QRS complexes can be reliably detected in the far-field electrocardiogram.

9. The implantable cardiac stimulator according to one of Claims 1 to 8, **characterised in that** the additional detection arrangement or far-field electrocardiogram evaluation unit (480) thereof is configured to detect a QRS complex in the far-field electrocardiogram by means of a pattern comparison.

10. The implantable cardiac stimulator according to one of Claims 1 to 9, **characterised in that** the far-field electrocardiogram evaluation unit (480) is configured to subject a far-field electrocardiogram to a signal form analysis in order to determine signal peaks that are to be assigned to ventricular events.

11. The implantable cardiac stimulator according to one of Claims 1 to 8, **characterised in that** the far-field electrocardiogram evaluation unit (480) is configured to detect sense events in the far-field electrocardiogram by comparison with a fixedly predefined or variable threshold value.

12. The implantable cardiac stimulator according to one of Claims 1 to 11, **characterised in that** the implantable cardiac stimulator has a memory (80) and is configured to store in the memory (80) information concerning a withheld defibrillation shock.

## Revendications

1. Stimulateur cardiaque tri-ventriculaire implantable (10) avec
- un boîtier (42) au moins partiellement électriquement conducteur, et
- un raccord de ligne d'électrode de défibrillation pour le raccordement d'une électrode de défibrillation ventriculaire (38, 40) au moyen d'une ligne d'électrode (16),
- un générateur de chocs de défibrillation (50) relié au raccord de ligne d'électrode de défibrillation et conçu pour générer un choc de défibrillation et délivrer celui-ci par le biais du raccord de ligne d'électrode de défibrillation,
- une unité de détection du ventricule droit (58) reliée ou destinée à être reliée à au moins une électrode de détection du ventricule droit (18, 20) présentant une surface relativement petite par rapport à l'électrode de défibrillation (38, 40), par le biais d'une ligne d'électrode du ventricule droit (16, 16'), et conçue pour enregistrer et traiter des potentiels électriques du myocarde ventriculaire,
- une unité de détection du ventricule gauche (66) reliée ou destinée à être reliée à au moins une électrode de détection du ventricule gauche (32, 34) par le biais d'une ligne d'électrode du ventricule gauche, et conçue pour enregistrer et traiter des potentiels électriques du myocarde ventriculaire,
- une unité de détection de l'oreillette droite (62) reliée ou destinée à être reliée à au moins une électrode de détection de l'oreillette droite (22, 24) par le biais d'une ligne d'électrode de l'oreillette droite (14), et conçue pour enregistrer et traiter des potentiels électriques du myocarde atrial,
- une unité de commande (54 ; 54') reliée à l'unité de détection du ventricule droit (58) et au générateur de chocs de défibrillation (50), et conçue pour détecter une fibrillation ventriculaire et pour actionner le générateur de chocs de défibrillation (50) en fonction du résultat de détection, pour la délivrance d'un choc de défibrillation,
- un dispositif de détection supplémentaire (280 ; 460, 470) pour la perception d'évènements ventriculaires et atriaux, travaillant indépendamment de l'au moins une électrode de détection du ventricule droit,
dans lequel l'unité de commande (54 ; 54') est reliée au dispositif de détection supplémentaire (280 ; 460, 470) et conçue pour inhiber la délivrance d'un choc de défibrillation lorsque le dispositif de détection supplémentaire (280 ; 460, 470) décèle de manière fiable un rythme normal,
**caractérisé en ce que**
l'unité d'évaluation (280) est conçue pour évaluer un rythme comme étant un rythme normal lorsqu'un seul évènement du ventricule gauche est perçu suite à un évènement atrial intrinsèque ou stimulé dans une fenêtre temporelle prédéfinie « attente de temps AV », et lorsque l'intervalle entre des évènements consécutifs du ventricule gauche correspond en outre à l'intervalle des évènements atriaux précédents, augmenté d'une tolérance.

2. Stimulateur cardiaque implantable selon la revendication 1, **caractérisé en ce que** la fenêtre temporelle prédéfinie « attente de temps AV » pour la perception ventriculaire gauche suite à des évènements atriaux peut être réglée manuellement.

3. Stimulateur cardiaque implantable selon l'une des revendications 1 à 2, **caractérisé en ce que** le dispositif de détection supplémentaire (460, 470) est conçu pour la dérivation d'un électrocardiogramme à champ lointain, et relié ou destiné à être relié à deux électrodes présentant une surface relativement grande en comparaison avec une paire d'électrodes de détection bipolaires relativement éloignées l'une de l'autre.

4. Stimulateur cardiaque implantable selon l'une des revendications 1 à 3, avec un boîtier (42) au moins partiellement électriquement conducteur, **caractérisé en ce que** le dispositif de détection supplémentaire comporte
- une unité de détection d'électrocardiogramme à champ lointain (460, 470), laquelle comporte une première entrée reliée à un raccord pour une électrode de détection ventriculaire ainsi qu'une deuxième entrée reliée au boîtier (42) électriquement conducteur, et laquelle est conçue pour générer un électrocardiogramme à champ lointain sur la base des potentiels électriques appliqués aux deux entrées,
et
- une unité d'évaluation d'électrocardiogramme à champ lointain (480) reliée à l'unité de détection d'électrocardiogramme (460, 470) et conçue pour détecter des caractéristiques de signaux caractérisant des dépolarisations ventriculaires dans un électrocardiogramme à champ lointain généré par l'unité de détection d'électrocardiogramme à champ lointain (460, 470).

5. Stimulateur cardiaque implantable selon la revendication 4, **caractérisé en ce que** le raccord pour une électrode de défibrillation ventriculaire pour le raccordement d'une ligne d'électrode ventriculaire (16') avec deux bobines de choc, notamment une bobine de choc proximale (40) et une bobine de choc distale (38), est conçu de telle manière que les bobines de choc sont à relier indépendamment l'une de l'autre à l'unité de détection d'électrocardiogramme à champ lointain (460, 470).

6. Stimulateur cardiaque implantable selon la revendication 1, **caractérisé en ce que** le dispositif de détection supplémentaire comporte une unité de détection d'électrocardiogramme à champ lointain (460, 470)
reliée ou destinée à être reliée à une ligne d'électrode ventriculaire (16') avec deux bobines de choc, et conçu pour dériver un électrocardiogramme à champ lointain entre les deux bobines de choc,
et
- une unité d'évaluation d'électrocardiogramme à champ lointain (480) reliée à l'unité de détection d'électrocardiogramme à champ lointain (460, 470) et conçue pour détecter des caractéristiques de signaux caractérisant des dépolarisations ventriculaires dans un électrocardiogramme à champ lointain généré par l'unité de détection d'électrocardiogramme à champ lointain (460, 470).

7. Stimulateur cardiaque implantable selon l'une des revendications 1 à 4, avec un boîtier (42) au moins partiellement électriquement conducteur, **caractérisé en ce que** le dispositif de détection supplémentaire comporte
- une unité de détection d'électrocardiogramme à champ lointain (460, 470) reliée ou destinée à être reliée d'une part à une électrode atriale et d'autre part au boîtier électriquement conducteur, et conçue pour dériver un électrocardiogramme à champ lointain entre ces deux électrodes,
et
- une unité d'évaluation d'électrocardiogramme à champ lointain (480) reliée à l'unité de détection d'électrocardiogramme à champ lointain (460, 470) et conçue pour détecter des caractéristiques de signaux caractérisant des dépolarisations ventriculaires dans un électrocardiogramme à champ lointain généré par l'unité de détection d'électrocardiogramme à champ lointain (460, 470).

8. Stimulateur cardiaque implantable selon l'une des revendications 1 à 7, **caractérisé en ce que** l'unité d'évaluation (480) est de préférence conçue pour évaluer un rythme comme étant un rythme normal lorsque des complexes QRS peuvent être décelés de manière fiable dans l'électrocardiogramme à champ lointain.

9. Stimulateur cardiaque implantable selon l'une des revendications 1 à 8, **caractérisé en ce que** le dispositif de détection supplémentaire ou son unité d'évaluation d'électrocardiogramme à champ lointain (480) est conçu(e) pour détecter un complexe QRS dans l'électrocardiogramme à champ lointain au moyen d'une comparaison de motifs.

10. Stimulateur cardiaque implantable selon l'une des revendications 1 à 9, **caractérisé en ce que** l'unité d'évaluation d'électrocardiogramme à champ lointain (480) est conçue pour soumettre un électrocardiogramme à champ lointain à une analyse de forme de signal pour déterminer des pics de signaux attribuables à des évènements ventriculaires.

11. Stimulateur cardiaque implantable selon l'une des revendications 1 à 8, **caractérisé en ce que** l'unité d'évaluation d'électrocardiogramme à champ lointain (480) est conçue pour détecter des évènements de détection dans l'électrocardiogramme à champ lointain par une comparaison avec une valeur seuil fixement prédéfinie ou variable.

12. Stimulateur cardiaque implantable selon l'une des revendications 1 à 11, **caractérisé en ce que** le stimulateur cardiaque implantable comporte une mémoire (80) et est conçu pour enregistrer dans la mémoire (80) des informations concernant un choc de défibrillation retenu.
